(19) Europäisches Patentamt
European Patent Office
Office européen des brevets

(11) Veröffentlichungsnummer: **0 279 366 B1**

# (12) EUROPÄISCHE PATENTSCHRIFT

(45) Veröffentlichungstag der Patentschrift: **12.08.92**

(51) Int. Cl.[5]: **C07D 239/52**, C07D 239/56

(21) Anmeldenummer: **88102056.4**

(22) Anmeldetag: **12.02.88**

Die Akte enthält technische Angaben, die nach dem Eingang der Anmeldung eingereicht wurden und die nicht in dieser Patentschrift enthalten sind.

## (54) Verfahren zur Herstellung von Pyrimidinen.

(30) Priorität: **18.02.87 DE 3705084**

(43) Veröffentlichungstag der Anmeldung:
**24.08.88 Patentblatt 88/34**

(45) Bekanntmachung des Hinweises auf die Patenterteilung:
**12.08.92 Patentblatt 92/33**

(84) Benannte Vertragsstaaten:
**CH DE FR GB IT LI NL**

(56) Entgegenhaltungen:
**JP-A-61 134 378**
**US-A- 4 287 343**
**US-A- 4 492 598**

(73) Patentinhaber: **HOECHST AKTIENGESELLSCHAFT**
**Postfach 80 03 20**
**W-6230 Frankfurt am Main 80(DE)**

(72) Erfinder: **Lachhein, Stephen, Dr.**
**Kiedricher Strasse 18**
**W-6238 Hofheim am Taunus(DE)**
Erfinder: **Ressel, Hans-Joachim**
**Lorsbacher Strasse 17**
**W-6234 Hattersheim am Main(DE)**

EP 0 279 366 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Verfahren zur Herstellung von Pyrimidinen der Formel I

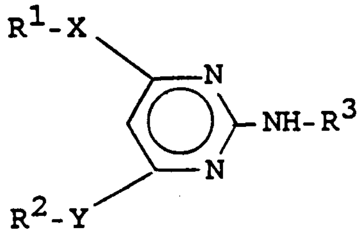

I,

worin

| | |
|---|---|
| X und Y | unabhängig voneinander Sauerstoff oder Schwefel und |
| $R^1$ und $R^2$ | unabhängig voneinander $(C_1\text{-}C_4)$Alkyl, $(C_1\text{-}C_4)$Alkoxy$(C_1\text{-}C_2)$alkyl oder Halo$(C_1\text{-}C_4)$alkyl bedeuten und |
| $R^3$ | H oder einen Rest $-COR^4$ bedeutet, wobei $R^4$ für $(C_1\text{-}C_4)$Alkyl, Halo$(C_1\text{-}C_4)$alkyl, $(C_1\text{-}C_4)$-Alkoxy oder Halo$(C_1\text{-}C_4)$alkoxy oder Phenyl oder Phenoxy, die beide ein-bis dreifach durch Halogen, $(C_1\text{-}C_4)$Alkoxy oder $(C_1\text{-}C_4)$Alkyl substituiert sein können, steht, |

dadurch gekennzeichnet, daß man ein Propandiimidat der Formel II

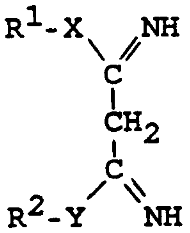

II

worin $R^1$ und $R^2$ wie bei Formel I definiert sind,
oder eines seiner Salze mit einem Kohlensäureiminderivat der Formel III

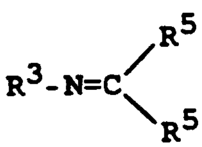

III,

worin

| | |
|---|---|
| $R^3$ | wie bei Formel I definiert ist und |
| $R^5$ | unabhangig voneinander Halogen, $(C_1\text{-}C_4)$Alkoxy, $(C_1\text{-}C_4)$Alkylthio, Phenoxy oder Phenylthio bedeutet, in einem inerten Lösungsmittel in Gegenwart einer Base umsetzt. |

Verbindungen der Formel I sind wertvolle Zwischenprodukte bei der Herstellung von Sulfonylharnstoffen mit herbizider Wirkung (US-PS 4.169.719, EP-A 071 958 (US-A-4,492,598)).

Bekannt ist ein Verfahren zur Herstellung der Verbindungen I im Falle $R^3$ = H bei dem diese Verbindungen durch Umsetzung von Propandiimidaten mit wäßriger Cyanamidlösung oder Chlorcyan in einem Zwei- oder Dreistufenprozeß hergestellt werden (EP-A 0 024 200). Die Bildung und anschließende Isolierung eines N-Cyanoimidates als Zwischenprodukt sind charakteristische Verfahrensmerkmale dieses Prozesses.

Ferner ist gemäß JP-A 61/134378 ein Verfahren zur Herstellung von Pyrimidinen mit Alkoxycarbonylamino-Resten beschrieben, bei welchem Propandiimidate mit Alkoxycarbonylisothiocyanat umgesetzt werden.

Überraschenderweise verläuft das erfindungsgemäße Verfahren als verfahrenstechnisch einfacher Einstufenprozeß ohne isolierbare Zwischenprodukte und liefert direkt die Verbindungen I in hoher Ausbeute. Nebenprodukte werden nur in untergeordnetem Maße gebildet. Nach Beendigung der Reaktion und anschließender Filtration verbleibt das Endprodukt in hoher Reinheit.

Das erfindungsgemäße Verfahren wird zweckmäßigerweise so durchgeführt, daß man das Propandiimidat der Formel II, dessen Mono- oder Disalz isoliert, in Lösung oder als Suspension mit einer Verbindung der Formel III bei Reaktionstemperaturen von -20 bis 100 °C, vorzugsweise -20 bis 60 °C, in Gegenwart einer Base umsetzt.

Als Salze des Propandiimidates werden bevorzugt solche der Fluor-, Chlor- oder Bromwasserstoffsäure, der Schwefel-oder Phosphorsäure eingesetzt. Haloalkylreste für $R^1$, $R^2$, $R^3$ sind beispielsweise $CH_2Cl$, $CHCl_2$, $CCl_3$, $CH_2CH_2Cl$ oder $CH_2CF_3$. Bevorzugte Bedeutung für X, Y ist Sauerstoff, für $R^1$ und $R^2$ ($C_1$-$C_4$)-Alkyl, insbesondere Methyl, für $R^3$ ein Wasserstoffatom oder ein Rest -$COR^4$, worin $R^4$ ($C_1$-$C_4$)Alkyl oder ($C_1$-$C_4$)Alkoxy, insbesondere ($C_1$-$C_4$)Alkyl, bedeutet, und für $R^5$ jeweils ($C_1$-$C_4$)Alkoxy, ($C_1$-$C_4$)Alkylthio oder insbesondere Chlor, wobei in dem Falle, in welchem $R^5$ jeweils Chloratome sind, $R^3$ vorzugsweise kein Wasserstoffatom ist.

Als inerte Lösungsmittel sind solche geeignet, die unter den jeweiligen Reaktionsbedingungen inert sind. Beispielsweise können als Lösungsmittel Wasser, aliphatische Alkohole mit 1 bis 4 C-Atomen, wie Methanol und Ethanol, aliphatische Ketone, wie Aceton und Methylisobutylketon, halogenierte aliphatische Kohlenwasserstoffe mit 1 bis 3 C-Atomen, wie Methylenchlorid und Chloroform, aliphatische und cycloaliphatische Ether, wie Diethylether, Dioxan und Tetrahydrofuran, aliphatische Ester mit 3 bis 6 C-Atomen, wie Methylacetat, Ethylacetat und Butylacetat, aromatische und cycloaliphatische Kohlenwasserstoffe mit 5 oder 6 Ringatomen, wie Toluol, Xylol, Hexan und Cyclohexan, aliphatische Nitrile, wie Acetonitril, oder deren Mischungen verwendet werden.

Als Basen können die Verbindung der Formel II selbst oder anorganische Basen wie Alkali- oder Erdalkalihydroxide, -carbonate, -hydrogencarbonate oder -alkoholate oder organische Basen, z.B. stickstoffhaltige Basen wie Trialkylamine, Pyridin etc. eingesetzt werden.

Die Verbindungen der Formel II können nach bekannten Methoden hergestellt werden (S.M. McElvain and I.D. Schroeder, J. Amer. Chem. Soc. 71, 40 (1949); B. Harstun, DE-A 2 426 913). Das Monosalz und das Bisimidat dieser Verbindungen können aus dem entsprechenden Disalz durch Umsetzung mit Basen wie Alkali- und Erdalkalihydroxiden, -carbonaten, -hydrogencarbonaten oder -alkoholaten in einem inerten Lösungsmittel hergestellt werden.

Die Verbindungen der Formel III können nach bekannten Methoden hergestellt werden, (s. z.B. Houben Weyl, Methoden der organischen Chemie, Band E4, S. 522 ff, 580 ff; DE-A 1 932 297 (GB-A-1,256,834)).

Um störende Einflüsse von Sauerstoff auf die Reaktion zu vermeiden, ist es zweckmäßig, unter Inertgasatmosphäre, beispielsweise unter Stickstoff, zu arbeiten.

Nachfolgende Beispiele sollen das erfindungsgemäße Verfahren näher erläutern:

**Verfahrensbeispiele**

Beispiel 1

4,6-Dimethoxy-2-(methoxycarbonylamino)-pyrimidin

Bei -10 °C werden unter Stickstoffüberlagerung 13 g (0,1 mol) Dimethyl 1,3-propandiimidat und 13,8 g (0,1 mol) pulverisiertes trockenes Kaliumcarbonat in 150 ml Tetrahydrofuran eingetragen. Bei gleicher Temperatur tropft man dann 15,6 g (0,1 mol) Methoxycarbonylisocyaniddichlorid zu. Man rührt noch für eine Stunde bei 0 °C, dann für vier Stunden bei Raumtemperatur.

Zur Aufarbeitung wird das Reaktionsgemisch auf Wasser gegossen und mit Ethylacetat extrahiert. Die organische Phase wird neutral gewaschen und über $Na_2SO_4$ getrocknet. Nach Abdampfen des Lösungsmittels im Vakuum verbleibt ein gelbliches Oel, das beim Abkühlen erstarrt.
Ausbeute: 19,6 g (92 % d. Th.)
Fp.: 92-94 °C

Beispiel 2

2-Acetylamino-4,6-dimethoxypyrimidin

Analog Beispiel I wird Dimethyl 1,3-propandiimidat mit 14,0 g (0,1 mol) Acetylisocyaniddichlorid

umgesetzt.
Ausbeute: 17,3 g (88 % d. Th.) leicht gelbliche Krist.
Fp.: 38-39 °C

Beispiel 3

2-Amino-4,6-dimethoxypyrimidin

Bei -20 °C werden unter Stickstoffüberlagerung 13,0 g (0,1 mol) Dimethyl 1,3-propandiimidat und 13,8 g (0,1 mol) pulverisiertes trockenes Kaliumcarbonat in 150 ml Acetonitril (getrocknet) eingetragen. Bei gleicher Temperatur tropft man dann wahrend 30 min. 8,9 g (0,1 mol) Carbonimidsäuredimethylester gemischt mit 20 ml trockenem Acetonitril zu. Anschließend wird bei 0 °C für 1 Stunde und bei Raumtemperatur für vier Sturden gerührt.

Das Reaktionsgemisch wird auf Wasser gegossen und mit Ethylacetat extrahiert. Die organische Phase wird neutral gewaschen, getrocknet über $Na_2SO_4$ und am Rotationsverdampfer eingedampft.

Es verbleibt ein gelblicher Feststoff, der im Vakuum teilweise zu weißen Kristallen sublimiert.
Ausbeute: 13,6 g (87,8 % d. Th.)
Fp. 93-94 °C

Beispiel 4

2-Amino-4,6-diethoxypyrimidin

15,8 g (0,1 mol) Diethyl 1,3-propandiimidat und 8,4 g (0,1 mol) Natriumhydrogencarbonat werden bei -10 °C in trockenem Acetonitril (150 ml) unter Rühren und Stickstoffüberlagerung vorgelegt. Man tropft bei gleicher Temperatur 16,3 g (0,1 mol) N-Acetylcarbonimidsäuredithiodimethylester, gemischt mit 20 ml trockenem Acetonitril zu. Danach läßt man das Gemisch langsam auf Raumtemperatur erwärmen und rührt für 5 Stunden nach.

Das Lösungsmittel wird am Rotationsverdampfer im Vakuum abdestilliert, der Rückstand (2-Acetylamino-4,6-diethoxypyrimidin) mit einer Lösung von 7,0 g (0,11 mol) KOH (88 %ig) in 200 ml Methanol für 10 Stunden am Rückfluß erhitzt.

Anschließend wird das Methanol im Vakuum abdestilliert, der Rückstand in Wasser aufgenommen und mit Methylenchlorid extrahiert. Die Extrakte werden am Rotationsverdampfer eindestilliert.

Verbleibendes Rohprodukt (2-Amino-4,6-diethoxypyrimidin)
15,6 g (85 % Ausbeute)
Fp. 95 °C

Beispiel 5

2-Ethoxycarbonylamino-4,6-dimethoxypyrimidin

Bei -20 °C werden unter Stickstoff-Atmosphäre 10,15 g (50 mmol) 1,3-Dimethylpropanbisimidat-Dihydrochlorid und 16,8 g (200 mmol) $NaHCO_3$ in 100 ml Methylenchlorid suspendiert und mit 20 ml Wasser versetzt.

Während die Temperatur auf -5 °C ansteigt, entweicht die halbe molare Menge $CO_2$. Dann tropft man bei -10 bis -5 °C 8,6 g (50 mmol) Ethoxycarbonylisocyaniddichlorid gemischt mit 20 ml Methylenchlorid zu und läßt anschließend die Temperatur für ca. 1 Std. bei -5 bis 0 °C. Hierbei bildet sich das offenkettige Zwischenprodukt, das im Dünnschichtchromatogramm ($CH_2Cl_2/CH_3OH$ = 95/5) als polarer Fleck zu erkennen ist.
Anschließend wird langsam bis zum Rückfluß (40 °C) erwärmt. Es wird der zweite Anteil $CO_2$ entwickelt. Die Cyclisierung zum gewünschten Pyrimidin-Derivat kann man im DC erkennen. Man läßt für 1,5 Std. bei 40 °C und arbeitet dann auf, indem in $CH_2Cl_2//H_2O$ verteilt wird, die org. Phase nochmals mit Wasser gewaschen und nach Trocknen über $Na_2SO_4$ eingedampft wird.
Das Rohprodukt wird über eine dünne Kieselgelschicht mit $CH_2Cl_2$ eluiert, um polare Verunreinigungen zu entfernnen. Die Ausbeute beträgt 89 % d. Th., bez. auf das eingesetzte Isocyaniddichlorid.

Beispiel 6

2-Ethoxycarbonylamino-4,6-dimethoxypyrimidin

Bei -20 °C werden unter Stickstoff-Atmosphäre 10,15 g (50 mmol) 1,3-Dimethylpropanbisimidat-Monohydrochlorid und 12,6 g (150 mmol) $NaHCO_3$ in 100 ml Methylisobutylketon (MIBK) suspendiert und mit 20 ml Wasser versetzt.
Während die Temperatur auf -5 °C ansteigt, entweicht $CO_2$. Dann läßt man bei -5 °C 8,6 g (50 mmol) Ethoxycarbonylisocyaniddichlorid, gemischt mit 20 ml MIBK, zutropfen und hält anschließend die Temperatur für ca. 1 Std. bei -5 bis 0 °C. Hierbei bildet sich das offenkettige Zwischenprodukt, das im DC ($CH_2Cl_2/CH_3OH$ = 95/5) als polarer Fleck zu erkennen ist.
Anschließend wird langsam bis zum Rückfluß (40 °C) erwärmt. Es wird erneut $CO_2$ entwickelt. Die Cyclisierung zum gewünschten Pyrimidin-Derivat kann man im Dünnschichtchromatogramm erkennen. Man läßt für 1,5 Std. bei 40 °C und arbeitet dann auf, indem in MIBK $H_2O$ verteilt wird, die org. Phase nochmals mit Wasser gewaschen und nach Trocknen über $Na_2SO_4$ eingedampft wird.
Das Rohprodukt wird über eine dünne Kieselgelschicht mit $CH_2Cl_2$ eluiert, um polare Verunreinigungen zu entfernen. Die Ausbeute beträgt 88 % d. Th., bezogen auf das eingesetzte Isocyaniddichlorid.

Analog zu den in den Beispielen 1-6 beschriebenen Verfahrensweisen lassen sich durch Reaktion von Verbindungen der Formel II, in denen $R^1$, $R^2$, X und Y die in der folgenden Tabelle genannten Bedeutungen haben, mit Verbindungen der Formel III, in denen $R^3$ und $R^5$ die in der folgenden Tabelle definierten Bedeutungen haben, weitere Verbindungen der Formel I mit den in der Tabelle definierten Bedeutungen für $R^1$, $R^2$, $R^3$, X und Y herstellen:

| Bsp. | $R^1$ | $R^2$ | X | Y | $R^3$ | $R^5$ |
|---|---|---|---|---|---|---|
| 7 | $C_2H_5$ | $C_2H_5$ | O | O | $COCH_3$ | Cl |
| 8 | $C_2H_5$ | $C_2H_5$ | O | O | $COCH_3$ | Cl |
| 9 | $CH_3$ | $CH_3$ | O | O | $COOC_6H_5$ | Cl |
| 10 | $C_2H_5$ | $C_2H_5$ | O | O | $COOC_6H_5$ | Cl |
| 11 | $CH_3$ | $CH_3$ | O | O | $COCH_2Cl$ | $SC_2H_5$ |
| 12 | $CH_3$ | $CH_3$ | O | O | $COCHCl_2$ | Cl |
| 13 | $CH_3$ | $CH_3$ | O | O | $COCCl_3$ | Cl |
| 14 | $C_2H_5$ | $C_2H_5$ | S | S | $COOCH_3$ | $OC_2H_5$ |
| 15 | $CH(CH_3)_2$ | $CH(CH_3)_2$ | O | O | $COOCH_3$ | Cl |
| 16 | $CH_3$ | $CH_3$ | O | S | $COOCH_3$ | Cl |
| 17 | $C_2H_5$ | $C_2H_5$ | O | S | $COOCH_3$ | Cl |
| 18 | $CH_3$ | $CH_3$ | O | S | $COC_2H_5$ | $SCH_3$ |
| 19 | $C_2H_5$ | $C_2H_5$ | O | O | $COOC_2H_5$ | Cl |
| 20 | $CH_2Cl$ | $CH_2Cl$ | O | O | $COOCH_3$ | Cl |
| 21 | $CH_2Cl$ | $CH_2Cl$ | O | O | $COC_2H_5$ | Cl |
| 22 | $CH_2Cl$ | $CH_2Cl$ | O | O | $COOC_6H_5$ | Cl |
| 23 | $CH_2Cl$ | $CH_2Cl$ | S | S | $COOCH_2Cl$ | $SC_6H_5$ |
| 24 | $CF_3$ | $CF_3$ | O | O | $COOCH_3$ | $SC_6H_5$ |
| 25 | $CF_3$ | $CF_3$ | O | O | $COOC_2H_5$ | $OC_6H_5$ |
| 26 | $CF_3$ | $CF_3$ | S | S | $COOC_2H_5$ | Cl |
| 27 | $CH_2OCH_3$ | $CH_2OCH_3$ | O | O | $COOCH_3$ | Cl |
| 28 | $CH_2OC_2H_5$ | $CH_2OC_2H_5$ | O | S | $COOCH_3$ | Cl |
| 29 | $CH_2OC_2H_5$ | $CH_2OCH_3$ | O | O | $COOC_2H_5$ | Cl |

## Patentansprüche

1. Verfahren zur Herstellung von Verbindungen der Formel I

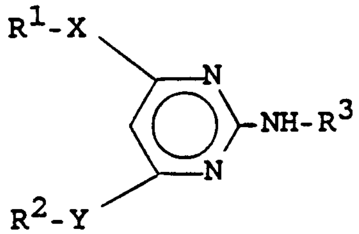

I,

worin

X und Y Sauerstoff oder Schwefel und

$R^1$ und $R^2$ unabhängig voneinander $(C_1\text{-}C_4)$Alkyl, $(C_1\text{-}C_4)$Alkoxy-$(C_1\text{-}C_2)$alkyl oder Halo$(C_1\text{-}C_4)$-alkyl bedeuten und

$R^3$ H oder einen Rest $-COR^4$ bedeutet, wobei $R^4$ für $(C_1\text{-}C_4)$Alkyl, Halo$(C_1\text{-}C_4)$alkyl, $(C_1\text{-}C_4)$Alkoxy oder Halo$(C_1\text{-}C_4)$alkoxy oder Phenyl oder Phenoxy, die beide ein- bis dreifach durch Halogen, $(C_1\text{-}C_4)$Alkoxy oder $(C_1\text{-}C_4)$Alkyl substituiert sein können, steht,

dadurch gekennzeichnet, daß man ein Propandiimidat der Formel II,

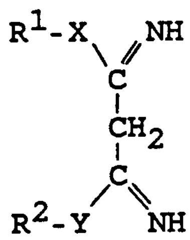

II

worin $R^1$ und $R^2$ wie bei Formel I definiert sind,
oder eines seiner Salze mit einem Kohlensäureiminderivat der Formel III

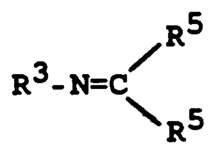

III,

worin

$R^3$ wie bei Formel I definiert ist und

$R^5$ unabhängig voneinander Halogen, $(C_1\text{-}C_4)$Alkoxy, $(C_1\text{-}C_4)$Alkylthio, Phenoxy oder Phenylthio bedeutet, in einem inerten Lösungsmittel in Gegenwart einer Base umsetzt.

**2.** Verfahren gemäß Anspruch 1, dadurch gekennzeichnet, daß X und Y Sauerstoff sowie $R^1$ und $R^2$ unabhängig voneinander $(C_1\text{-}C_4)$Alkyl bedeuten.

**3.** Verfahren gemäß Anspruch 1 oder 2, dadurch gekennzeichnet, daß $R^3$ ein Wasserstoffatom oder einen Rest $-COR^4$, worin
$R^4$ $(C_1\text{-}C_4)$Alkyl oder $(C_1\text{-}C_4)$Alkoxy bedeutet, und
$R^5$ jeweils $(C_1\text{-}C_4)$-Alkoxy oder $(C_1\text{-}C_4)$-Alkylthio bedeuten.

**4.** Verfahren gemäß Anspruch 1 oder 2, dadurch gekennzeichnet, daß $R^3$ einen Rest der Formel $-COR^4$, worin
$R^4$ $(C_1\text{-}C_4)$Alkyl oder $(C_1\text{-}C_4)$Alkoxy bedeutet, und
$R^5$ jeweils Chlor bedeuten.

**5.** Verfahren gemäß einem oder mehreren der Ansprüche 1 bis 4, dadurch gekennzeichnet, daß man als Basen Alkali- oder Erdalkalihydroxide, -carbonate, -hydrogencarbonate oder -alkoholate, Trialkylamine, Pyridin oder die Verbindung der Formel II selbst einsetzt.

**6.** Verfahren gemäß einem oder mehreren der Ansprüchen 1 bis 5, dadurch gekennzeichnet, daß man als inertes Lösungsmittel eines aus der Gruppe, enthaltend aliphatische Alkohole mit 1 bis 4 C-Atomen, aliphatische Ketone, aliphatische und cycloaliphatische Ether, halogenierte aliphatische Kohlenwasserstoffe mit 1 bis 3 C-Atomen, aliphatische Ester mit insgesamt 3 bis 6 C-Atomen, aliphatische Kohlenwasserstoffe mit 5 bis 12 C-Atomen, aromatische und cycloaliphatische Kohlenwasserstoffe mit 5 oder 6 Ringatomen, aliphatische Nitrile mit 1 bis 4 C-Atomen und Mischungen aus mindestens 2 der aufgeführten Lösungsmittel, verwendet.

**7.** Verfahren nach Anspruch 6, dadurch gekennzeichnet, daß als inertes Lösungsmittel eines aus der Gruppe, enthaltend Methanol, Ethanol, Aceton, Methylisobutylketon, Diethylether, Dioxan, Tethrahydrofuran, Methylenchlorid, Chloroform, Essigsäurealkylester mit 1 bis 4 C-Atomen im Alkylrest, Hexan, Toluol, Xylole, Cyclohexan und Acetonitril, verwendet.

**8.** Verfahren nach einem oder mehreren der Ansprüche 1 bis 7, dadurch gekennzeichnet, daß man bei einer Reaktionstemperatur von -20 bis 100 °C arbeitet.

**9.** Verfahren nach Anspruch 8, dadurch gekennzeichnet, daß man bei -20 bis 60 °C arbeitet.

**10.** Verfahren nach einem oder mehreren der Ansprüche 1 bis 9, dadurch gekennzeichnet, daß man das Verfahren unter Inertgasatmosphäre durchführt.

**Claims**

**1.** A process for the preparation of compounds of the formula I

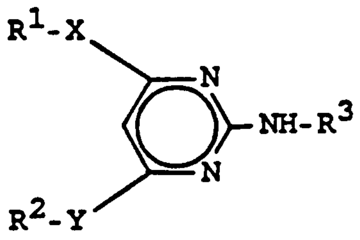

I

in which

X and Y independently of one another denote oxygen or sulfur and

$R^1$ and $R^2$ independently of one another denote $(C_1$-$C_4)$-alkyl, $(C_1$-$C_4)$alkoxy-$(C_1$-$C_2)$alkyl or halogeno-$(C_1$-$C_4)$alkyl and

$R^3$ denotes H or a radical $-COR^4$ in which $R^4$ denotes $(C_1$-$C_4)$alkyl, halogeno-$(C_1$-$C_4)$alkyl, $(C_1$-$C_4)$-alkoxy, halogeno-$(C_1$-$C_4)$-alkoxy, phenyl or phenoxy the latter both of which are unsubstituted or monosubstituted to trisubstituted by halogen, $(C_1$-$C_4)$alkoxy or $(C_1$-$C_4)$-alkyl,

which comprises reacting a propanediimidate of the formula II

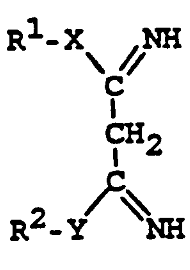

II

in which $R^1$ and $R^2$ are as defined in formula I, or one of its salts,
in an inert solvent and in the presence of a base, with a carbonic acid imine derivative of the formula III

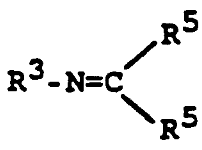

III

in which

$R^3$ is as defined in formula I and the

$R^5$s independently of one another denote halogen, $(C_1\text{-}C_4)$alkoxy, $(C_1\text{-}C_4)$alkylthio, phenoxy or phenylthio.

**2.** The process as claimed in claim 1, wherein X and Y denote oxygen and $R^1$ and $R^2$ independently of one another denote $(C_1\text{-}C_4)$alkyl.

**3.** The process as claimed in claim 1 or 2 wherein $R^3$ denotes a hydrogen atom or a $-COR^4$ radical in which $R^4$ denotes $(C_1\text{-}C_4)$alkyl or $(C_1\text{-}C_4)$alkoxy, and each of the $R^5$s denotes $(C_1\text{-}C_4)$alkoxy or $(C_1\text{-}C_4)$-alkylthio.

**4.** The process as claimed in claim 1 or 2 wherein $R^3$ denotes a radical of the formula $-COR^4$ in which $R^4$ denotes $(C_1\text{-}C_4)$alkyl or $(C_1\text{-}C_4)$alkoxy, and each of the $R^5$s denotes chlorine.

**5.** The process as claimed in one or more of claims 1 to 4, wherein the bases employed are alkali or alkaline earth metal hydroxides, carbonates, bicarbonates or alcoholates, trialkylamines, pyridine or the compound of the formula II itself.

**6.** The process as claimed in one or more of claims 1 to 5, wherein the inert solvent used is a solvent belonging to the group containing aliphatic alcohols having 1 to 4 carbon atoms, aliphatic ketones, aliphatic and cycloaliphatic ethers, halogenated aliphatic hydrocarbons having 1 to 3 carbon atoms, aliphatic esters having a total of 3 to 6 carbon atoms, aliphatic hydrocarbons having 5 to 12 carbon atoms, aromatic and cycloaliphatic hydrocarbons having 5 or 6 ring atoms, aliphatic nitriles having 1 to 4 carbon atoms, and mixtures of at least 2 of the solvents mentioned.

**7.** The process as claimed in claim 6, wherein the inert solvent used is a solvent belonging to the group containing methanol, ethanol, acetone, methyl isobutyl ketone, diethyl ether, dioxane, tetrahydrofuran, methylene chloride, chloroform, alkyl acetates having 1 to 4 carbon atoms in the alkyl radical, hexane, toluene, xylenes, cyclohexane and acetonitrile.

**8.** The process as claimed in one or more of claims 1 to 7, wherein the reaction is carried out at a temperature from -20 to 100 °C.

**9.** The process as claimed in claim 8, wherein the reaction is carried out at -20 to 60 °C.

**10.** The process as claimed in one or more of claims 1 to 9, wherein the process is carried out under an inert gas atmosphere.

## Revendications

**1.** Procédé pour préparer des composés répondant à la Formule I :

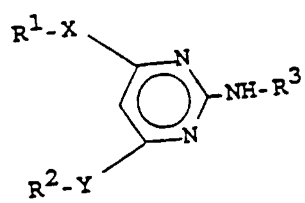

I,

dans laquelle

X et Y représentent chacun, l'oxygène ou le soufre,

$R^1$ et $R^2$ représentent chacun, indépendamment l'un de l'autre, un alkyle en $C_1\text{-}C_4$, un $(C_1\text{-}C_4)$-alcoxy-$(C_1\text{-}C_2)$alkyle ou un halogéno-alkyle en $C_1\text{-}C_4$ et

$R^3$ représente l'hydrogène ou un radical $-COR^4$ dans lequel $R^4$ représente un alkyle en $C_1\text{-}C_4$, un halogéno-alkyle en $C_1\text{-}C_4$, un alcoxy en $C_1\text{-}C_4$, un halogéno-alcoxy en $C_1\text{-}C_4$, un

phényle ou un phénoxy, chacun de ces deux derniers pouvant porter de un à trois substituants pris dans l'ensemble constitué par les halogènes, les alcoxy en $C_1$-$C_4$ et les alkyles en $C_1$-$C_4$,

procédé caractérisé en ce qu'on fait réagir un propanediimidate répondant à la Formule II :

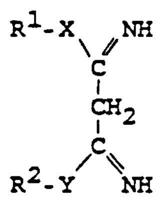

II

dans laquelle $R^1$ et $R^2$ ont les significations qui leur ont été données à propos de la formule I, ou l'un de ses sels, avec un dérivé de l'acide carbonimidique répondant à la Formule III :

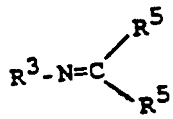

III,

dans laquelle

$R^3$ a la signification qui lui a été donnée à propos de la Formule I et les

$R^5$ représentent chacun, indépendamment l'un de l'autre, un halogène, un alcoxy en $C_1$-$C_4$, un alkylthio en $C_1$-$C_4$, un phénoxy ou un phénylthio,

dans un solvant inerte, en présence d'une base.

**2.** Procédé selon la revendication 1 caractérisé en ce que X et Y représentent chacun l'oxygène et $R^1$ et $R^2$ représentent chacun, indépendamment l'un de l'autre, un alkyle en $C_1$-$C_4$.

**3.** Procédé selon l'une des revendications 1 et 2, caractérisé en ce que $R^3$ représente un atome d'hydrogène ou un radical -$COR^4$ dans lequel $R^4$ représente un alkyle en $C_1$-$C_4$ ou un alcoxy en $C_1$-$C_4$, et les $R^5$ représentent chacun un alcoxy en $C_1$-$C_4$ ou un alkylthio en $C_1$-$C_4$.

**4.** Procédé selon l'une des revendications 1 et 2, caractérisé en ce que $R^3$ représente un radical -$COR^4$ dans lequel R4 représente un alkyle en $C_1$-$C_4$ ou un alcoxy en $C_1$-$C_4$, et les $R^5$ représentent chacun le chlore.

**5.** Procédé selon une ou plusieurs des revendications 1 à 4, caractérisé en ce qu'on utilise, comme bases, des hydroxydes, carbonates, hydrogénocarbonates ou alcoolates de métaux alcalins ou de métaux alcalino-terreux, des trialkylamines, la pyridine ou le composé de Formule II lui même.

**6.** Procédé selon une ou plusieurs des revendications 1 à 5, caractérisé en ce qu'on utilise, comme solvant inerte, un solvant pris dans l'ensemble constitué par les alcools aliphatiques contenant de 1 à 4 atomes de carbone, les cétones aliphatiques, les éthers aliphatiques et cycloaliphatiques, les hydrocarbures aliphatiques halogénés contenant de 1 à 3 atomes de carbone, les esters aliphatiques contenant au total de 3 à 16 atomes de carbone, les hydrocarbures aliphatiques contenant de 5 à 12 atomes de carbone, les hydrocarbures aromatiques et cycloaliphatiques contenant 5 ou 6 atomes dans le cycle, les nitriles aliphatiques contenant de 1 à 4 atomes de carbone et les mélanges d'au moins deux des solvants cités.

**7.** Procédé selon la revendication 6 caractérisé en ce qu'on utilise, comme solvant inerte, un solvant pris dans l'ensemble constitué par le méthanol, l'éthanol, l'acétone, la méthyl-isobutyl-cétone, l'oxyde de diéthyle, le dioxanne, le tétrahydrofuranne, le chlorure de méthylène, le chloroforme, les acétates d'alkyles contenant de 1 à 4 atomes de carbone dans leur radical alkyle, l'hexane, le toluène, les

xylènes, le cyclohexane et l'acétonitrile.

**8.** Procédé selon une ou plusieurs des revendications 1 à 7, caractérisé en ce qu'on opère à une température réactionnelle comprise entre -20 et 100 ° C.

**9.** Procédé selon la revendication 8 caractérisé en ce qu'on opère à une température comprise entre -20 et 60 ° C.

**10.** procédé selon une ou plusieurs des revendications 1 à 9, caractérisé en ce qu'on effectue le procédé sous un gaz inerte.